# EUROPEAN PATENT APPLICATION

(11) **EP 4 130 740 A1**
(43) Date of publication of application: **08.02.2023**
(21) Application number: 21776675.7
(22) Date of filing: 24.03.2021
(51) Int. Cl.: G01N 33/53, G01N 33/543

(54) **MEASURING METHOD FOR FRAGMENT INCLUDING 7S-DOMAIN OF HUMAN TYPE-IV COLLAGEN, AND KIT TO BE USED THEREFOR**

(30) Priority: 25.03.2020 JP 2020055142
(71) Applicant: Fujirebio Inc., Shinjuku-ku Tokyo 163-0410 (JP)
(72) Inventor: KATAGIRI Noriko, Hachioji-shi, Tokyo 192-0031 (JP); YAGI Shintaro, Hachioji-shi, Tokyo 192-0031 (JP); AOYAGI Katsumi, Hachioji-shi, Tokyo 192-0031 (JP)
(74) Representative: Müller-Boré & Partner Patentanwälte PartG mbB
(86) International application number: PCT/JP2021/012389
(87) International publication number: WO 2021/193763

(57) **Abstract**

The present invention provides a measurement method comprising: measuring a fragment containing a human type IV collagen 7S domain in a sample by sandwich immunoassay, using a capture antibody in which a first monoclonal antibody capable of binding specifically to the human type IV collagen 7S domain is immobilized on a carrier and a labeled antibody in which a second monoclonal antibody capable of binding specifically to the human type IV collagen 7S domain binds to a labeling substance.

## Description

### [Technical Field]

The present invention relates to a method for measuring a fragment containing a human type IV collagen 7S domain in a sample and a kit for use therein.

### [Background Art]

Type IV collagen is a major protein that constitutes basement membranes together with laminins, and its structural unit is a trimer having a helical structure consisting of three α-chains. Although the subunits constituting the trimer differ depending on the tissue, in many tissues including the liver, the trimer is composed of two α1-chains and one α2-chain. A type IV collagen molecule has a structure specific to the type IV collagen molecule at both ends of the TH domain (Triple Helical collagenous domain) forming the helical structure, and the N-terminal structure is called a 7S domain and the C-terminal structure is called an NC1 domain (Non-collagenous domain). The C-terminal NC1 domain forms a dimer with the NC1 domain of another type IV collagen molecule, and the N-terminal 7S domain forms a tetramer with the 7S domain of another type IV collagen molecule. All of these multimers are tightly bound by covalent bonds, and by polymerization of these, the type IV collagen molecule forms a network structure (Chicken-wire network).

In the human liver, liver fibrosis gradually progresses to liver cirrhosis and liver cancer due to prolonged type B and type C viral chronic hepatitis and alcoholic chronic hepatitis, progressive non-alcoholic steatohepatitis (NASH), and the like, and at that time, it is known that type IV collagen increases. The amount of type IV collagen leaked from the basement membrane into the blood increases with the progress of such liver fibrosis, so that the blood concentration of the components derived from the type IV collagen molecule is an index of the progress of liver fibrosis. In particular, the 7S domain, which is strongly bound by covalent bonds, is considered to be less susceptible to proteolytic enzymes and stable in blood, and it is used as a biomarker for diagnosing the degree of progression (severity) of liver fibrosis and liver cirrhosis.

However, the only such 7S domain measuring reagent approved as a diagnostic agent is the "Type IV Collagen 7S Kit (manufactured by DENIS Pharma K.K.)" which is a diagnostic agent using a radioimmunoassay (RIA). Type IV Collagen 7S Kit is a reagent using an anti-human type IV collagen rabbit polyclonal antibody.

Further, Japanese Unexamined Patent Application Publication No. Hei 2-1553 (PTL 1) describes a method in which a monoclonal antibody that cross-reacts with the pepsin-solubilized human type IV collagen 7S domain is used as an enzyme-labeled antibody, and a monoclonal antibody that reacts only with the human type IV collagen 7S domain is bound to a solid phase carrier to form an antibody-bound solid phase carrier (capture antibody), and the human type IV collagen 7S domain is quantified by a one-step sandwich immunoassay.

### [Citation List]

### [Patent Literature]

[PTL 1] Japanese Unexamined Patent Application Publication No. Hei 2-1553

### [Summary of Invention]

### [Technical Problem]

However, since Type IV Collagen 7S Kit described above uses a polyclonal antibody against human type IV collagen and is based on the principle of inhibition of binding competition, the measurement accuracy is still insufficient. The present inventors have found that there is in particular a problem that the measured value in a specimen derived from a healthy person becomes high and the false positive rate becomes high, which makes it unsuitable for screening and differentiating patients in the early stages of liver fibrosis.

The present inventors have also found the following. In the blood derived from patients with liver fibrosis (such as liver cirrhosis patients and NASH patients), collagen fragments of various forms, from small to large molecules, are present as type IV collagen fragments containing the 7S domain. Nonetheless, in the method of Type IV Collagen 7S Kit described above and in the method described in PTL 1, a monoclonal antibody that cross-reacts with a pepsin-solubilized human type IV collagen 7S domain is used as an enzyme-labeled antibody, it is impossible to sufficiently detect small molecule collagen fragments, so that it is difficult to comprehensively measure all of these collagen fragments, which may reduce the measurement accuracy depending on the specimen.

Moreover, the present inventors have found that these conventional methods also have a problem that the measurement accuracy tends to decrease due to being easily affected by a site other than the 7S domain of type IV collagen and other components contained in the sample.

The present invention has been made in view of the problems newly discovered by the present inventors, and an object thereof is to provide a measurement method which makes it possible to reduce non-specificity for a sample derived from a healthy person (negative specimen) without reducing the specificity for a sample derived from a patient (positive specimen) and to measure fragments containing a human type IV collagen 7S domain in a sample with high accuracy, and a kit for use therein.

### [Solution to Problem]

As a result of earnest studies to solve the above problems, the present inventors have found that sandwich immunoassay combining a monoclonal antibody capable of site-specific binding to the human type IV collagen 7S domain makes it possible to sufficiently reduce the measured value of the sample derived from a healthy person (negative specimen) without reducing the measured value of the sample derived from the patient (positive specimen), and in particular, in low concentration ranges of the fragments containing the human type IV collagen 7S domain, higher measurement accuracy than before can be achieved. Further, it has been found that according to this method, as a type IV collagen fragment containing a 7S domain, any form of collagen fragment can be comprehensively measured, and moreover, it is possible to achieve higher measurement accuracy than before due to reduced influence of substances present in the sample and sites other than the 7S domain of type IV collagen. Thus, the present invention has been completed.

The aspects of the present invention obtained from such findings are as follows.
[1] A measurement method comprising: measuring a fragment containing a human type IV collagen 7S domain in a sample by sandwich immunoassay, using a capture antibody in which a first monoclonal antibody capable of binding specifically to the human type IV collagen 7S domain is immobilized on a carrier and a labeled antibody in which a second monoclonal antibody capable of binding specifically to the human type IV collagen 7S domain binds to a labeling substance.
[2] The measurement method according to [1], comprising:
   a capturing step of contacting the sample with the capture antibody to capture the fragment containing the human type IV collagen 7S domain using the capture antibody; and
   after the capturing step, a labeling step of contacting the labeled antibody with the fragment containing the human type IV collagen 7S domain captured by the capture antibody to label the fragment containing the human type IV collagen 7S domain captured by the capture antibody.
[3] The measurement method according to [1] or [2], wherein a salt concentration of a reaction system of the fragment containing the human type IV collagen 7S domain and the first monoclonal antibody is 0.35 M or more.
[4] A kit for use in the measurement method according to any one of [1] to [3], comprising:
   a first monoclonal antibody capable of binding specifically to the human type IV collagen 7S domain and a carrier capable of binding to the first monoclonal antibody; and
   a second monoclonal antibody capable of binding specifically to the human type IV collagen 7S domain and a labeling substance capable of binding to the second monoclonal antibody.
[5] The kit according to [4], comprising:
   a capture antibody in which the first monoclonal antibody is immobilized on the carrier; and
   a labeled antibody in which the second monoclonal antibody is bound to the labeling substance.

### [Advantageous Effects of Invention]

The present invention can provide a measurement method which makes it possible to reduce non-specificity for a sample derived from a healthy person (negative specimen) without reducing the specificity for a sample derived from a patient (positive specimen) and to measure fragments containing a human type IV collagen 7S domain in a sample with high accuracy, and a kit for use therein.

### [Brief Description of Drawings]

[Fig. 1] Fig. 1 is a picture of electrophoresis photograph showing the results of SDS-PAGE in the collagenase treatment of Example 1 (1).
[Fig. 2] Fig. 2 is a graph showing the relationship between the concentration and absorbance of each monoclonal antibody when a type IV collagen 7S fragment is used as an antigen.
[Fig. 3] Fig. 3 is a graph showing the relationship between the concentration and absorbance of each monoclonal antibody when a pepsin-solubilized type IV collagen fragment is used as an antigen.
[Fig. 4] Fig. 4 is a graph showing the relationship between the concentration and luminescence of type IV collagen 7S fragment in a standard solution.
[Fig. 5] Fig. 5 is a graph showing the relationship between the actual concentration of type IV collagen 7S fragment in a standard solution and the coefficient of variation of quantitative values.
[Fig. 6] Fig. 6 is a scatter plot showing the correlation between the measurement results of Example 2 (CL 7S) and the measurement results of Comparative Example 1 (RIA 7S).
[Fig. 7] Fig. 7 is a scatter plot showing the correlation between the measurement results of Example 2 (CL 7S) and the measurement results of Comparative Example 1 (RIA 7S) only for the healthy person groups.
[Fig. 8] Fig. 8 is a graph showing the distribution of measurement values of Example 3 ((b) CL 7S) and Comparative Example 2 ((a) RIA 7S) for the healthy person group 1.
[Fig. 9] Fig. 9 is a graph showing the distribution of measurement values of Example 3 ((b) CL 7S) and Comparative Example 2 ((a) RIA 7S) for the healthy person group 2.
[Fig. 10] Fig. 10 is a diagram showing the ROC curves of Example 2 (CL 7S) and Comparative Example 1 (RIA 7S) for the liver cirrhosis group.
[Fig. 11] Fig. 11 is a diagram showing the ROC curves of Example 2 (CL 7S) and Comparative Example 1 (RIA 7S) for the NASH group.
[Fig. 12] Fig. 12 is a graph showing the luminescence (a) of each fraction of the specimen LC58, the luminescence (b) of each fraction of the specimen LC51, and the luminescence (c) of each fraction of a healthy person specimen, when CIV09 antibody-immobilized magnetic particles are used.
[Fig. 13] Fig. 13 is a graph showing the luminescence (a) of each fraction of the specimen LC58 and the luminescence (b) of each fraction of the type IV collagen 7S fragment, when CIV09 antibody-immobilized magnetic particles or CIV03 antibody-immobilized magnetic particles are used.
[Fig. 14] Fig. 14 is a graph showing the luminescence of each fraction of the specimen LC51, when CIV09 antibody-immobilized magnetic particles or CIV03 antibody-immobilized magnetic particles are used.
[Fig. 15] Fig. 15 is a graph showing the quantitative values (pool recovery values) of each of the pools 1 to 6 in Fig. 14.

### [Description of Embodiments]

Hereinafter, the present invention is described in detail according to preferred embodiments thereof.

### <Method for measuring fragments containing human type IV collagen 7S domain>

The present invention provides a method for measuring a fragment containing a human type IV collagen 7S domain (hereinafter simply referred to as the "measurement method of the present invention" in some cases), comprising: measuring a fragment containing a human type IV collagen 7S domain in a sample by sandwich immunoassay, using a capture antibody in which a first monoclonal antibody capable of binding specifically to the human type IV collagen 7S domain is immobilized on a carrier and a labeled antibody in which a second monoclonal antibody capable of binding specifically to the human type IV collagen 7S domain binds to a labeling substance.

In the present invention, the "sample" is not particularly limited as long as it is a sample in which a fragment containing the human type IV collagen 7S domain can be present. Generally, a blood specimen collected from a diagnosis target (human) is used, and examples of the blood specimen include serum, plasma, and whole blood, preferably serum or plasma, and more preferably serum. Further, the sample may be appropriately diluted or suspended with the following diluted solution or pretreated, if necessary. Examples of the pretreatment include collagenase treatment.

### (Fragment containing human type IV collagen 7S domain)

In the sample (preferably a blood specimen), fragments of various forms of the human type IV collagen molecule, from small to large molecules, may be present as components derived from the human type IV collagen molecule. Examples of such fragments include a fragment composed of a 7S domain, which does not contain the TH domain and the NC1 domain (hereinafter referred to as the "type IV collagen 7S fragment" in some cases); a fragment composed of a TH domain, which does not contain the NC1 domain and the 7S domain; a fragment composed of an NC1 domain, which does not contain the TH domain and the 7S domain; a fragment containing two or more of each of these domains in whole or in part; and a complex of these fragments with other basement membrane components such as laminin. In the present invention, the "7S domain" includes both a 7S domain that does not form a multimer and a 7S domain that forms a dimer to a tetramer, the "TH domain" includes both a TH domain that does not form a helical structure and a TH domain that forms the helical structure, and the "NC1 domain" includes both an NC1 domain that does not form a multimer and an NC1 domain that forms a dimer.

In the present invention, the "type IV collagen 7S fragment" is preferably composed of a tetramer of the 7S domain, and indicates a fragment contained in a gel filtration fraction having a molecular weight corresponding to a tetramer of human type IV collagen 7S domain, which is a degradation product obtained by further collagenase treatment of pepsin-solubilized type IV collagen fragments solubilized by pepsin digestion, more specifically, a gel filtration fraction having a molecular weight of 200,000 (or a mass of 200 kDa) or more and around 400,000 (for example, 300 kDa to 440 kDa), or a fragment of human type IV collagen containing a 7S domain having a molecular weight corresponding thereto. The time for the collagenase treatment varies depending on the concentration of the collagenase to be added, but is preferably, for example, 30 minutes to 14 hours, and the treatment temperature is preferably 30 to 37°C. In the present invention, the "pepsin-solubilized type IV collagen fragment" is a human type IV collagen fragment solubilized by pepsin digestion, and indicates a mixture containing two or more of the following: a fragment composed of a 7S domain (type IV collagen 7S fragment), a fragment composed of a TH domain, a fragment composed of an NC1 domain, and a fragment containing two or more of each of these domains in whole or in part. The pepsin-solubilized type IV collagen fragment can be appropriately obtained by a conventionally known method. For example, it can be obtained by treating an extract containing human type IV collagen molecules from the human placenta with pepsin, and a commercially available one may be used.

In the present invention, the "fragment containing the human type IV collagen 7S domain" to be measured may be a fragment of human type IV collagen containing the 7S domain, and examples thereof include the above-described type IV collagen 7S fragment; a fragment composed of the 7S domain and all or part of the TH domain; and a fragment composed of the 7S domain, all or part of the TH domain, and the NC1 domain, and one of these may be used alone or two or more thereof may be used as a mixture. According to the measurement method of the present invention, measurement is even possible in both cases where the type IV collagen 7S fragments having a small molecular weight are contained and the case where fragments having a large molecular weight with the 7S domain possibly not sufficiently exposed are contained, as the fragments containing the human type IV collagen 7S domain. Therefore, it is possible to measure fragments containing a human type IV collagen 7S domain with higher accuracy than the conventional methods.

### (Monoclonal Antibody)

In the present invention, the "first monoclonal antibody capable of binding specifically to the human type IV collagen 7S domain" and the "second monoclonal antibody capable of binding specifically to the human type IV collagen 7S domain" (hereinafter collectively referred to as "anti-7S domain antibody") are monoclonal antibodies capable of specifically recognizing human type IV collagen 7S domain only and binding thereto. That is, the anti-7S domain antibody does not bind to a site other than the 7S domain of human type IV collagen. It can be confirmed that an antibody is the anti-7S domain antibody, for example, by being able to specifically recognize and bind to the type IV collagen 7S fragment; or by being able to bind to the molecules contained in the fractions corresponding to the molecular weight of the human type IV collagen 7S domain (preferably tetramer) among the gel filtration fractions of the sample and being able to bind not only to type IV collagen fragments containing the TH domain and NC1 domain (such as pepsin-solubilized type IV collagen fragments solubilized by pepsin treatment) but also to the type IV collagen 7S fragments.

Note that the "antibody" in the present invention includes not only complete antibodies but also antibody fragments (such as Fab, Fab', F(ab')₂, Fv, single chain antibodies, and diabodies) or low molecular weight antibodies in which antibody variable regions are bound to each other.

The anti-7S domain antibody according to the present invention can be produced by appropriately employing and improving a conventionally known method, and it can be produced, for example, by a method for producing a monoclonal antibody using a cell (hybridoma) having an antibody-producing cell and a myeloma cell fused together (typically, the method by Koehler and Millstein (Kohler & Milstein, Nature, 256: 495, 1975)).

Examples of the antibody-producing cells include spleen cells, lymph node cells, and peripheral blood leukocytes of animals (such as mice, rats, hamsters, rabbits, monkeys, goats, sheep, donkeys, camels, alpaca, and chickens) immunized with an immunogen, and antibody-producing cells obtained by allowing an immunogen to act in a medium against the above-mentioned cells or lymphocytes previously isolated from a non-immunized animal can also be used.

In the present invention, the immunogen is not particularly limited as long as it is a fragment containing the human type IV collagen 7S domain, and examples thereof include the type IV collagen 7S fragment, the pepsin-solubilized type IV collagen fragment, and a fragment obtained by partially digesting the pepsin-solubilized type IV collagen with collagenase (corresponding to the "collagenase partially digested type IV collagen fragment" in Examples).

As the myeloma cell, various known cell lines can be used. The antibody-producing cells and the myeloma cells may be of different animal species origin as long as they can be fused, but are preferably of the same animal species origin.

The hybridoma is obtained, for example, by cell fusion between mouse myeloma cells and spleen cells obtained from mice immunized with the immunogen. From among the many hybridomas obtained, hybridomas are screened that produce monoclonal antibodies highly reactive with the type IV collagen 7S fragment. In addition, if necessary, the epitopes of the monoclonal antibodies produced by the selected hybridomas are further analyzed from among them to identify the clones that produce the monoclonal antibodies that bind to the type IV collagen 7S fragment. This makes it possible to obtain hybridomas that produce monoclonal antibodies capable of specifically biding to the human type IV collagen 7S domain (anti-7S domain antibody). Such monoclonal antibodies can be obtained by culturing these hybridomas or from the ascites of the mammal administered with the hybridomas.

Further, for example, if a DNA encoding an anti-7S domain antibody can be obtained, it can also be produced by a recombinant DNA method. Examples of the recombinant DNA method include a method in which a DNA encoding the antibody is cloned from a hybridoma, the antibody-producing cell, or the like, incorporated into an appropriate vector, introduced into a host cell (such as a mammalian cell line, E. coli, yeast cell, insect cell, and or plant cell), and produced as a recombinant antibody (for example, P. J. Delves, Antibody Production: Essential Techniques, 1997, WILEY; P. Shepherd and C. Dean Monoclonal Antibodies, 2000, OXFORD UNIVERSITY PRESS; Vandamme A. M. et al., Eur. J. Biochem. 192: 767-775, 1990). In the expression of the DNA encoding the above antibody, DNA encoding a heavy chain or a light chain may be separately incorporated into an expression vector to transform a host cell, or DNA encoding heavy and light chains may be integrated into a single expression vector to transform a host cell (for example, the method described in International Publication No. WO94/11523).

The recombinant antibody can be obtained in a substantially pure and uniform form by culturing the host cell and separating and purifying it in the cultured host cell or from the culture solution. For the separation and purification of the recombinant antibody, the method used in the usual purification of a polypeptide can be used. When a transgenic animal production technique is used to produce a transgenic animal having an antibody gene integrated therein (such as cow, goat, sheep, or pig), it is also possible to obtain a large amount of monoclonal antibody, derived from an antibody gene, from the milk of that transgenic animal.

In the present invention, the first monoclonal antibody and the second monoclonal antibody may be the same as or different from each other. When the sample is a blood specimen, the human type IV collagen 7S domain usually forms a dimer to a tetramer (preferably a tetramer) in the blood specimen. For this reason, even when the first monoclonal antibody and the second monoclonal antibody are antibodies of the same type that recognize and bind to the same site, each of them can bind to the human type IV collagen 7S domain of any of the multimers.

### (Capture Antibody)

In the measurement method of the present invention, as a capture antibody that captures the fragment containing the human type IV collagen 7S domain, a capture antibody in which the first monoclonal antibody is immobilized on a carrier is used.

In the present invention, the "carrier" is not particularly limited as long as it can immobilize and carry the first monoclonal antibody. The material of such a carrier is not particularly limited as long as it is generally used for immunoassay, and examples thereof include high polymers (such as polystyrene, (meth)acrylic acid ester, polymethylmethacrylate, polyimide, and nylon), gelatin, cellulose, nitrocellulose, glass, latex, silica, metals (such as gold, platinum, iron, cobalt, and nickel), metal compounds (such as iron oxide, cobalt oxide, and nickel ferrite), and composites and alloys thereof. Further, the carrier may be surface-modified with, for example, one or more active groups of a carboxy group, an epoxy group, a tosyl group, an amino group, a hydroxy group, an isothiocyanate group, an isocyanate group, an azide group, an aldehyde group, a carbonate group, an allyl group, an aminooxy group, a maleimide group, a thiol group, an NHS ester (N-hydroxy ester) group.

Further, in the present invention, the shape of the carrier is not particularly limited, and may be, for example, a plate, a fiber, a film, particles, or the like, but is preferably a particle from the viewpoint that the density of the first monoclonal antibody to be immobilized is easy to control and tends to be uniform. Further, the particles are preferably magnetic particles from the viewpoint of automation and shortening of time.

When the carrier is particles, the size thereof is not particularly limited, but the particle diameter is preferably in the range of 0.01 to 100 µm, and more preferably in the range of 0.1 to 10 um. When the particle diameter of the particles is less than the lower limit, magnetism and time required for magnetic collection tend to increase in the case of magnetic particles. Meanwhile, when the upper limit is exceeded, the specific surface area of the particles tends to be small, so that the amount of the first monoclonal antibody bound to the particles tends to be small.

As such particles, conventionally known particles can be appropriately used. For example, known particles such as magnetic particles described in Japanese Unexamined Patent Application Publication No. Hei 3-115862 (ferrite-coated particles, carboxylated ferrite particles), or commercially available particles such as Dynabeads (manufactured by Thermo Fisher), Magnosphere (manufactured by JSR Corporation), and Magrapid (manufactured by Sanyo Chemical Industries, Ltd.) can be appropriately used. Further, as those particles, one of these may be used alone or two or more thereof may be used in combination.

In the method for producing a capture antibody according to the present invention, as a method for immobilizing the first monoclonal antibody on the carrier, a conventionally known method or a method similar thereto can be appropriately employed, and it may be directly immobilized or indirectly immobilized on the surface of the carrier. A method for directly binding is, for example, a method in which the active group is added to the carrier, or a carrier having any of the above active groups is used, to bond the first monoclonal antibody by covalent bonding of the active group.

In the method of indirect immobilization, for example, by immobilizing a substance that binds to the first monoclonal antibody on the surface of the carrier and binding the first monoclonal antibody to the substance, the first monoclonal antibody can be indirectly immobilized on the surface of the carrier. The substance that binds to the first monoclonal antibody is not particularly limited, and examples thereof include a secondary antibody capable of binding to the first monoclonal antibody, protein G, protein A, a linker such as a linker molecule having the active group, and the like. Further, if the first monoclonal antibody is biotinylated or avidinylated (or streptavidinylated), the carrier may be avidinylated (or streptavidinylated) or biotinylated to immobilize the antibody by the interaction of biotin with avidin (or streptavidin).

Further, as to the capture antibody according to the measurement method of the present invention, a first monoclonal antibody and a carrier capable of binding to the first monoclonal antibody may be used in advance to produce a capture antibody in which the first monoclonal antibody is directly or indirectly immobilized on the carrier, followed by sandwich immunoassay. Alternatively, the first monoclonal antibody and the carrier capable of binding to the first monoclonal antibody may be separately provided in a reaction system of sandwich immunoassay, followed by immobilization of the first monoclonal antibody on the carrier in the same reaction system, or a complex of the first monoclonal antibody and a fragment containing the human type IV collagen 7S domain may be formed, followed by immobilization of then the first monoclonal antibody on the carrier. In this case, the combination of "the first monoclonal antibody and the carrier capable of binding to the first monoclonal antibody" includes any combination of the first monoclonal antibody and the carrier described above. When these are separately supplied to a reaction system, as described in the method of indirectly immobilizing the first monoclonal antibody on the carrier, a combination of the first monoclonal antibody and a carrier immobilized with a substance capable of binding to the first monoclonal antibody is preferable, or a combination of the biotinylated or avidinylated (or streptavidinylated) first monoclonal antibody with an avidinylated (or streptavidinylated) or biotinylated carrier is preferable.

The capture antibody according to the present invention contains at least the first monoclonal antibody and the carrier, but may also contain the linker, blocking agent, and the like, if necessary.

### (Labeled Antibody)

In the measurement method of the present invention, as a labeled antibody for labeling a fragment containing the human type IV collagen 7S domain, a labeled antibody obtained by binding a second monoclonal antibody to a labeling substance is used.

In the present invention, the "labeling substance" is not particularly limited as long as it can be detected by binding to a second monoclonal antibody. Examples thereof include enzymes; luminescent substances such as acridinium derivatives; fluorescent substances such as europium; fluorescent proteins such as allophycocyanin (APC) and phycoerythrin (R-PE); radioactive substances such as ¹²⁵I; low molecular weight labeling substances such as fluorescein isothiocyanate (FITC) and rhodamine isothiocyanate (RITC); gold particles; avidin; biotin; latex; dinitrophenyl (DNP); and digoxigenin (DIG). Among these, the labeling substance according to the present invention is preferably an enzyme and a luminescent substance, more preferably an enzyme, from the viewpoint that it tends to enable simple and stable tests and that it is possible to carry out simple, rapid, and highly sensitive measurements. Examples of the enzyme include, but are not limited to, horseradish peroxidase (HRP), alkaline phosphatase (ALP), β-galactosidase (β-gal), glucose oxidase, and luciferase.

In the method for producing a labeled antibody according to the present invention, as a method for binding the labeling substance to the second monoclonal antibody, a conventionally known method or a method similar thereto can be appropriately employed, and may be directly bonded or indirectly bonded. Examples of such a binding method include the same methods as those mentioned as the binding method between the carrier and the first monoclonal antibody in the capture antibody according to the present invention.

Further, as to the labeled antibody according to the measurement method of the present invention, a second monoclonal antibody and a labeling substance capable of binding to the second monoclonal antibody may be used in advance to produce a labeled antibody in which the second monoclonal antibody is directly or indirectly bound to the labeling substance, followed by sandwich immunoassay. Alternatively, the second monoclonal antibody and the labeling substance capable of binding to the second monoclonal antibody may be separately provided in a reaction system of sandwich immunoassay, followed by binding of the second monoclonal antibody on the labeling substance in the same reaction system, or a complex of the second monoclonal antibody and a fragment containing the human type IV collagen 7S domain may be formed, followed by binding of then the second monoclonal antibody on the labeling substance. In this case, the combination of "the second monoclonal antibody and the labeling substance capable of binding to the second monoclonal antibody" includes any combination of the second monoclonal antibody and the labeling substance described above. When these are separately supplied to a reaction system, a combination of the second monoclonal antibody and a labeling substance bound with a substance capable of binding to the second monoclonal antibody is preferable, or a combination of the biotinylated or avidinylated (or streptavidinylated) second monoclonal antibody with an avidinylated (or streptavidinylated) or biotinylated labeling substance is preferable. Examples of the substance that binds to the second monoclonal antibody include the same substances as those that bind to the first monoclonal antibody.

The labeled antibody according to the present invention contains at least the second monoclonal antibody and the labeling substance, but may also contain the linker, blocking agent, and the like, if necessary.

### (Sandwich Immunoassay)

The measurement method of the present invention measures a fragment containing a human type IV collagen 7S domain in a sample by sandwich immunoassay using the capture antibody and the labeled antibody.

In the present invention, "measurement" includes detection of extracting the presence or absence and amount of a target substance in a sample as a signal, as well as quantification or semi-quantification of the amount of the target substance. In the present invention, it is preferable that the measurement of the target substance is carried out by detecting a signal generated by the labeling substance and quantifying it as necessary. The "signal" includes coloration (color development), reflected light, luminescence, fluorescence, radiation by a radioisotope, and the like, and also includes not only those that can be confirmed with the naked eye but also those that can be confirmed by a measuring method or device according to the type of signal.

The "sandwich immunoassay" is a method in which the target substance ("fragment containing human type IV collagen 7S domain" in the present invention) is captured by an antibody for capture ("capture antibody" in the present invention) immobilized on a solid phase, the target substance is recognized by an antibody for detection ("labeled antibody" in the present invention), bound with the labeling substance, to form a complex of a capture antibody-target substance-labeled antibody, which is washed if necessary, and detection is carried out according to the type of the labeling substance.

Note that in the measurement method of the present invention, the process in which "the target substance ("fragment containing human type IV collagen 7S domain" in the present invention) is captured by an antibody for capture ("capture antibody" in the present invention) immobilized on a solid phase" includes forming a complex of a first antibody ("first monoclonal antibody" in the present invention) not immobilized on a solid phase ("carrier" in the present invention) and the target substance, or a complex of a first antibody not immobilized on a solid phase, the target substance, and the labeled antibody (or the second antibody described later) and then immobilizing the first antibody having the complex formed therein on the solid phase to allow the solid phase to capture the target substance (or the target substance and the labeled antibody) via the first antibody. In this case, the combination of the first antibody and the solid phase is the same as the combination described for "the first monoclonal antibody and the carrier capable of binding to the first monoclonal antibody" of the above-mentioned capture antibody, including its preferable embodiments.

Further, in the measurement method of the present invention, the process in which "the target substance is recognized by an antibody for detection ("labeled antibody" in the present invention), bound with the labeling substance" includes forming a complex of a second antibody ("second monoclonal antibody" in the present invention) not bound to a labeling substance and the target substance, or a complex of a second antibody not bound to a labeling substance, the target substance, and the capture antibody (or the first antibody described above) and then binding the labeling substance and the second antibody having the complex formed therein to allow the labeling substance to recognize the target substance (or the target substance and the capture antibody) via the second antibody. In this case, the combination of the second antibody and the labeling substance is the same as the combination described for "the second monoclonal antibody and the labeling substance capable of binding to the second monoclonal antibody" of the above-mentioned labeled antibody, including its preferable embodiments.

Examples of such sandwich immunoassay include the sandwich CLIA method, which is a form of chemiluminescent immunoassay (CLIA method), the sandwich CLEIA method, which is a form of chemiluminescent enzyme immunoassay (CLEIA method), the IRMA method (immunoradiation quantification method), which is a form of radioimmunoassay (RIA method), the sandwich ECLIA method, which is a form of electrochemiluminescence immunoassay (ECLIA method), and the sandwich FIA method, which is a form of fluorescent immunoassay (FIA method).

In the measuring method of the present invention, the salt concentration of the reaction system of the fragment containing the human type IV collagen 7S domain and the first monoclonal antibody (preferably a reaction system that uses the capture antibody to capture a fragment containing the human type IV collagen 7S domain) is preferably 0.35 M (M: mol/L, the same applies hereinafter) or more. The salt concentration is more preferably 0.35 to 1.3 M, more preferably 0.42 to 1.0 M, and further preferably 0.43 to 0.99 M. In the measurement method of the present invention, in particular, when the salt concentration is within the above range, non-specific reactions in the specimen derived from a healthy person can be further reduced, and the measuring accuracy can be further improved.

The present inventors have found the following. In the measurement of a fragment containing the human type IV collagen 7S domain, when the salt concentration at the time of binding to the first monoclonal antibody (preferably at the time of capturing using the capture antibody) is higher than the salt concentration in conventional sandwich immunoassay (for example, about 0.15 M), it is possible in particular to attenuate the binding to the human type IV collagen 7S domain contained in the sample derived from a healthy person, and to maintain and enhance the binding to the 7S domain contained in the sample derived from a patient with liver fibrosis (such as a liver cirrhosis patient or NASH patient). This would be partly because, as fragments containing the human type IV collagen 7S domain, the collagen fragment contained in the sample derived from a patient and the collagen fragment contained in the sample derived from a healthy person differed in composition, and according to the measurement method of the present invention, the non-specificity to the collagen fragment contained in the sample derived from a healthy person was reduced at a high salt concentration. Further, another reason would be because, in the samples, there was a wide variety of collagen fragments having different degrees of exposure of the 7S domain, including those having a large molecular weight, depending on the length of the TH domain and the like contained and the size of the collagen network structure, so that at low salt concentrations, a gap easily occurred between the measured value by the anti-7S domain antibody and the actual amount of the fragment containing the human type IV collagen 7S domain in the sample, whereas as high salt concentrations, the mask was removed and the 7S domain was exposed, solving the gap.

The reaction system of the fragment containing the human type IV collagen 7S domain and the first monoclonal antibody (preferably a reaction system for capturing with the capture antibody) is not particularly limited as long as it is a reaction system containing the sample, the first monoclonal antibody (preferably the capture antibody), and the salt, but is preferably an aqueous solution, and may further contain physiological saline, purified water; buffer solutions such as MES, Tris, CFB, MOPS, PIPES, HEPES, tricine buffer, bicine buffer, and glycine buffer; stabilizing proteins such as BSA; and various surfactants, and may further contain the labeled antibody if the reverse sandwich method or the one-step method is employed. Note that when a salt is contained in these additional components, the salt concentration of the reaction system also includes the concentration of the salt contained in these additional components.

When the sample contains salt, the salt concentration of the reaction system also includes the concentration of the salt contained in the sample. Blood specimens (whole blood, serum, plasma) generally contain a total of 140 mM to 150 mM worth of salt. If, for example, a blood specimen (such as whole blood, serum, or plasma) is used as the specimen, when 30 µL of a serum specimen as a sample is added to 50 µL of a particle (capture antibody) suspension containing a 0.6 M to 1.5 M salt as in the following Examples, the salt concentration of the reaction system for capturing the fragment containing the human type IV collagen 7S domain with the capture antibody is 0.43 M to 0.99 M.

The salt is not particularly limited, but is preferably at least one selected from the group consisting of alkali metal ions, inorganic salts of alkali metal ions, and organic salts of alkali metal ions, and examples of the inorganic salts of alkali metal ions include alkali metal chloride, alkali metal tartrate, alkali metal nitrate, and alkali metal sulfate. As the alkali metal ions, sodium ions and potassium ions are preferable. In this case, the salt concentration indicates a concentration corresponding to the concentration of alkali metal ions.

In such sandwich immunoassay, a first monoclonal antibody and a carrier capable of binding to the first monoclonal antibody (such as the capture antibody), the sample, and a second monoclonal antibody and a labeling substance capable of binding to the second monoclonal antibody (such as the labeled antibody) are brought into contact to finally form the complex (capture antibody-fragment containing human type IV collagen 7S domain (target substance)-labeled antibody). The contact method is not particularly limited, and a conventionally known method or a method similar thereto can be appropriately employed. Examples thereof include a method of adding to the sample a first monoclonal antibody and the carrier (such as the capture antibody) and/or a second monoclonal antibody and the labeling substance (such as the labeled antibody); and a method of adding the sample or the like to a solution of the capture antibody (such as a particle suspension when the carrier is particles) and/or a solution of the labeled antibody. Further, as the sample, one diluted or suspended with a diluted solution may be appropriately used.

When a first monoclonal antibody and a carrier capable of binding to the first monoclonal antibody (such as the capture antibody), the sample, and a second monoclonal antibody and a labeling substance capable of binding to the second monoclonal antibody (such as the labeled antibody) are brought into contact with each other, the salt concentration at the time of contacting the first monoclonal antibody (preferably the capture antibody) with the sample preferably satisfies the above conditions, but there are no other particular limitations. For example, in addition to the solvent of the capture antibody solution (such as a particle suspension solvent when the carrier is particles), the solvent of the labeled antibody solution, and the diluted solution, a reaction buffer may be further appropriately added. The solvent of the capture antibody solution, the solvent of the labeled antibody solution, the diluted solution, and the reaction buffer are not particularly limited, and examples thereof are, each independently, physiological saline, purified water; buffer solutions (such as sodium phosphate buffer, MES, Tris, CFB, MOPS, PIPES, HEPES, tricin buffer, bicine buffer, and glycine buffer), and may also be each independently added with a stabilizing protein such as BSA. Note that when these solutions contain a salt, the salt concentration of the reaction system also includes the concentration of the salt contained in the solutions. Note that when the sample contains a salt, the salt concentration of the reaction system also includes the concentration of the salt contained in the sample.

In the formation of the complex, the content of the first monoclonal antibody in the reaction system (in the case of a combination of two or more, the total amount thereof, the same applies hereinafter) is not particularly limited, and is not particularly limited because it is appropriately adjusted according to the type and concentration of the sample, the detection method, and the like, but is preferably, for example, 0.000001 to 0.01 w/v%, more preferably 0.00001 to 0.001 w/v%, from the viewpoint of efficient capture in a short time. In addition, the notation of "w/v%" in the present specification indicates weight/volume percent (g/100 mL).

Further, in the formation of the complex, the content of the second monoclonal antibody in the reaction system (in the case of a combination of two or more, the total amount thereof, the same shall apply hereinafter) is not particularly limited as well, and is not particularly limited because it is appropriately adjusted according to the type, concentration, detection method, and the like of the sample, but is preferably, for example, 0.000001 to 0.01 w/v%, more preferably 0.00001 to 0.001 w/v%, from the viewpoint of efficient labeling in a short time.

Further, the conditions for the contact are not particularly limited, and can be each independently and appropriately adjusted. For example, it can be carried out at 4 to 45°C, preferably 20 to 37°C; pH 6 to 9, preferably pH 6.5 to 8, for about 30 seconds to 12 hours, preferably about 1 minute to 1 hour, but the conditions are not limited to these.

Examples of the sandwich immunoassay include the forward sandwich method, which is a two-step method (method of sequentially carrying out capture by a carrier (preferably a reaction between the capture antibody and the fragment containing human type IV collagen 7S domain in the sample) and recognition by a labeling substance (preferably a reaction between the fragment containing human type IV collagen 7S domain bound to the capture antibody and the labeled antibody)), the reverse sandwich method (method in which the labeling substance carries out recognition in advance (preferably the labeled antibody is reacted with the fragment containing human type IV collagen 7S domain in the sample) and the generated complex is captured by a carrier (preferably reacted with a capture antibody)), and the one-step method (method in which the reaction of the capture antibody, the fragment containing human type IV collagen 7S domain in the sample, and the labeled antibody is simultaneously carried out in one step), and any of these can be employed.

Among these, as the measurement method of the present invention, the two-step method is preferable, and the forward sandwich method is more preferable, from the viewpoint of measurement accuracy. An aspect of the measurement method of the present invention which is such a forward sandwich method is, for example, a measuring method comprising:
a capturing step of contacting the sample with the capture antibody to capture the fragment containing the human type IV collagen 7S domain using the capture antibody; and
after the capturing step, a labeling step of contacting the labeled antibody with the fragment containing the human type IV collagen 7S domain captured by the capture antibody to label the fragment containing the human type IV collagen 7S domain captured by the capture antibody.

### [Capturing Step]

In the capturing step, the capture antibody is brought into contact with the sample, and the capture antibody is allowed to capture the fragment containing human type IV collagen 7S domain via the binding of the fragment containing human type IV collagen 7S domain to the first monoclonal antibody, that is, to form a first immune complex of the first monoclonal antibody and the fragment containing human type IV collagen 7S domain. The method for contacting the captured antibody with the sample and the conditions therefor are not particularly limited, but it is preferable to satisfy the conditions for the above salt concentration, and the methods and conditions described in the methods and conditions in which the capture antibody, the sample, and the labeled antibody are brought into contact with each other can be applied.

### [Washing Step]

The measurement method of the present invention preferably further includes, after the capturing step and before the following labeling step, a washing step of separating the fragment containing human type IV collagen 7S domain captured by the capture antibody and other impurities that are not bound (not captured) to the capture antibody to remove the impurities. The method for removing the impurities is not particularly limited, and a conventionally known method or a method similar thereto can be appropriately employed, and examples thereof include a method including recovering the captured antibody by centrifugation or magnetic collection after the capturing step to remove the liquid phase (supernatant). Further, in the washing step, the injection and removal of the washing solution may be repeated, if necessary. Examples of the washing solution include known neutral (preferably pH 6 to 9) buffer solutions (such as sodium phosphate buffer, MES, Tris, CFB, MOPS, PIPES, HEPES, tricine buffer, bicine buffer, and glycine buffer), or may be ones added with stabilizing proteins such as BSA or surfactants may be added.

### [labeling Step]

In the labeling step, the labeled antibody is brought into contact with the fragment containing human type IV collagen 7S domain captured by the capture antibody to label the fragment containing human type IV collagen 7S domain captured by the capture antibody, that is, to form a second immune complex of the second monoclonal antibody and the fragment containing human type IV collagen 7S domain captured by the capture antibody. The method for contacting the labeled antibody with the fragment containing human type IV collagen 7S domain captured by the capture antibody and the conditions therefor are not particularly limited, and the methods and conditions described in the methods and conditions in which the capture antibody, the sample, and the labeled antibody are brought into contact with each other can be applied.

The capturing step and the labeling step form the complex (second immune complex) containing the capture antibody-fragment containing human type IV collagen 7S domain-labeled antibody. After the labeling step, a washing step may be included, if necessary, in order to remove the labeled antibody or the like that did not form the complex. The washing step is the same as the above-mentioned washing step.

In the measurement method of the present invention, the labeling substance of the complex (capture antibody-fragment containing human type IV collagen 7S domain-labeled antibody) is measured by a predetermined method according to the labeling substance. For example, when the labeling substance of the labeled antibody is an enzyme, a color-developing substrate, a luminescent substrate, a chemiluminescent substrate, or the like corresponding to the enzyme is added, and signals (such as color-developing and luminescence) generated by reacting the enzyme with the substrate are detected. Thereby, the presence or absence and amount of the fragment containing human type IV collagen 7S domain in the sample can be detected as a signal. In addition, the amount of the fragment containing human type IV collagen 7S domain in the sample is generally quantified by comparison with the measured value with the standard solution containing the fragment containing human type IV collagen 7S domain at each concentration. The fragment containing human type IV collagen 7S domain used in the standard solution is not particularly limited, and examples thereof include the type IV collagen 7S fragment and the collagenase partially digested type IV collagen fragment. In this case, for example, when it is investigated at which position on the standard curve created based on the measured values by the standard solution, the measured values obtained for the sample are positioned, it is possible to determine the amount of the fragment containing the human type IV collagen 7S domain in the sample.

The measurement method of the present invention can be used for diagnosis of diseases associated with abnormal blood concentration of type IV collagen (evaluation of morbidity and its risk) and its assistance. Here, examples of the "diseases associated with abnormal blood concentration of type IV collagen" include diseases with the progression of liver fibrosis, and examples thereof include chronic viral hepatitis B and C, alcoholic hepatitis, non-alcoholic steatohepatitis (NASH), liver cirrhosis, and liver cancer.

In addition, the measurement method of the present invention can also be used for diagnosing or assisting diagnosing or differentiating the degree of progression (severity) of liver fibrosis, and screening patients who may have liver fibrosis. In particular, according to the measurement method of the present invention, it is possible to reduce the non-specificity for a sample derived from a healthy person (negative specimen) without reducing the specificity for a sample derived from a patient (positive specimen), as well as to achieve high measurement accuracy in low concentration ranges of a fragment containing human type IV collagen 7S domain. Therefore, it is useful as a measurement method for screening patients in the early stages of liver fibrosis from a healthy person group and for differentiating patients in the early stages of liver fibrosis from healthy subjects.

### <Kit for measuring fragments containing human type IV collagen 7S domain>

The present invention also provides a kit for measuring a fragment containing a human type IV collagen 7S domain (hereinafter simply referred to as the "kit of the present invention" in some cases), which is a kit for use in the measurement method of the present invention, comprising:
a first monoclonal antibody capable of binding specifically to the human type IV collagen 7S domain and a carrier capable of binding to the first monoclonal antibody; and
a second monoclonal antibody capable of binding specifically to the human type IV collagen 7S domain and a labeling substance capable of binding to the second monoclonal antibody. The kit of the present invention preferably comprises:
   a capture antibody in which the first monoclonal antibody is immobilized on the carrier; and
   a labeled antibody in which the second monoclonal antibody is bound to the labeling substance.

The first monoclonal antibody, the carrier capable of binding to the first monoclonal antibody, the second monoclonal antibody, the labeling substance capable of binding to the second monoclonal antibody, the capture antibody, and the labeled antibody, contained in the kit of the present invention, are as described in the measurement method of the present invention, including preferable embodiments thereof. Further, the first monoclonal antibody, the carrier capable of binding to the first monoclonal antibody, the second monoclonal antibody, the labeling substance capable of binding to the second monoclonal antibody, the capture antibody, and the labeled antibody, contained in the kit of the present invention, may be each independently in the form of solid (powder) or liquid dissolved in a buffer solution or the like.

The kit of the present invention may further include configurations that should be provided in conventional sandwich assays such as ELISA, CLEIA, ECLIA, CLIA, FIA, and immunochromatography. For example, it may include at least one selected from the group consisting of the standard solution (each concentration), control reagents, the diluted solution, the solvent of the captured antibody solution (such as particle suspension medium), the solvent of the labeled antibody solution, the reaction buffer, the washing solution, and dilution cartridges. Further, for example, when the labeling substance is an enzyme, it may further contain a substrate, a reaction terminator, and the like necessary for detecting and quantifying the labeling substance. Further, if necessary, a pretreatment liquid for pretreating the sample, an instruction manual for the kit, and the like may be further included.

### [Examples]

Hereinafter, the present invention is described in more detail based on Examples and Comparative Examples, but the present invention is not limited to the following Examples. In each Example and Comparative Example, the notation of "%" indicates weight/volume percent (w/v%: g/100 mL) unless otherwise specified.

### <Sandwich immunoassay using anti-type IV collagen 7S domain monoclonal antibody>

### (Example 1)

### (1) Collagenase Treatment

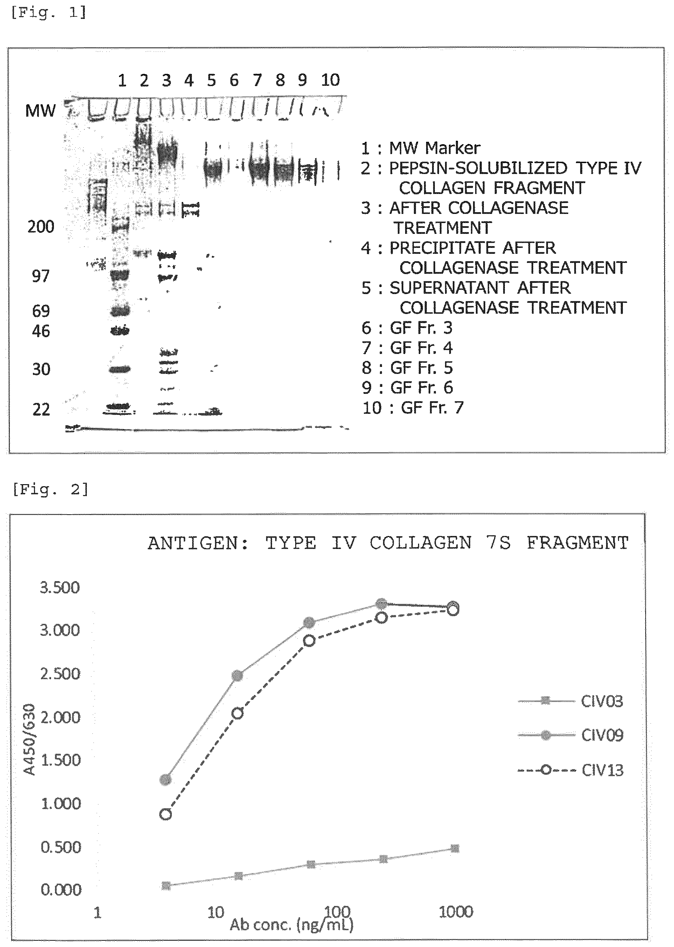

Type IV collagen 7S fragments were prepared based on the method of Risteli et al (Eur J Biochem, 108, p. 239-250). That is, first, pepsin-solubilized type IV collagen fragments (derived from human placenta, Sigma, C-7521) prepared by pepsin digestion were dissolved in 0.5 M acetic acid and dialyzed against 50 mM Tris (pH 7.2) containing 0.2 M NaCl and 2 mM CaCl₂. Then, collagenase (Collagenase "Amano", Wako, 607-19021) was added thereto, and the mixture was incubated at 37°C for 1 hour to further digest (collagenase treatment) the pepsin-solubilized type IV collagen fragments. The supernatant obtained by centrifuging the treatment liquid after the collagenase treatment was further separated by gel filtration. Superdex 200 pg 16/60 was used as the gel filtration column, and 1 mM EDTA 3 Na and PBS were used as the gel filtration buffers. Fig. 1 shows the results of SDS-PAGE (2 to 15% PAG Non-Reduce) for MW Marker (No. 1, molecular weight/1000); the pepsin-solubilized type IV collagen fragments (No. 2); the treatment solution after collagenase treatment (No. 3), precipitates thereof (No. 4), and supernatant (No. 5) thereof; and each fraction (fractions (GF Fr.) 3 to 7; No. 6 to 10). Fractions having molecular weights distributed at 250,000 (or a mass of 250 kDa) or more and in the vicinity of 400,000 (300 kDa to 440 kDa), corresponding to the molecular weight of the tetramer of the human type IV collagen 7S domain, were collected for use as the following type IV collagen 7S fragments.

In the present Examples and Comparative Examples, the "type IV collagen 7S fragments" are human type IV collagen fragments composed of a 7S domain and free of a TH domain, and were obtained by treating with collagenase and selecting the gel filtration fractions as above. In addition, as the immunogen below, collagenase partially digested type IV collagen fragments were used. The "collagenase partially digested type IV collagen fragments" are fragments prepared by digesting pepsin-solubilized type IV collagen fragments (manufactured by SIGMA) for 1 hour at a treatment temperature of 25°C using collagenase, with their main components having a molecular weight of 500 k or more, and are mainly a mixture of the type IV collagen 7S fragments and human type IV collagen fragments composed of the 7S domain and a part of the TH domain. Note that the above-mentioned "pepsin-solubilized type IV collagen fragments" are fragments prepared by solubilizing by pepsin digestion, and are a mixture of human type IV collagen fragments composed of the 7S domain, the TH domain, or the NC1 domain and human type IV collagen fragments containing two or more of each of them in whole or in part.

### (2) Preparation of monoclonal antibody

The "collagenase partially digested type IV collagen fragments" prepared in (1) above were used as an immunogen to prepare a monoclonal antibody. Specifically, a mixture of 10 µg (1 mg/mL) of collagenase partially digested type IV collagen fragments and an equal amount of complete Freund's adjuvant was intraperitoneally immunized to a BALB/c mouse twice with an interval of 2 weeks. In addition, abdominal immunization was carried out twice with an incomplete Freund's adjuvant with an interval of 2 weeks. Furthermore, 10 µg of collagenase partially digested type IV collagen fragments dissolved in PBS was administered intravenously to the tail vein as final immunization. On day 3 after the final immunization, the spleen was removed from this mouse, loosened into individual cells, and washed 3 times with RPMI-1640 medium. The mouse myeloma cell line Sp2/OAg14 in the logarithmic growth phase was washed 3 times with RPMI-1640 medium, mixed with the spleen cells, and fused. The resulting fusion cells were subjected to PEG removal by centrifugation (200 × g, 5 minutes), and then suspended in RPMI-1640 medium containing 10% fetal bovine serum as well as hypoxanthine, aminopterin, and thymidine (HAT) and seeded on 96-well cell culture plates. Only hybridomas were grown by culturing for about 10 days.

The pepsin-solubilized type IV collagen fragments and the type IV collagen 7S fragments prepared in (1) above were used as antigens, each diluted with PBS to 1 µg/mL, and dispensed into a 96-well plate (Nunc Maxisorp, 469914) at 100 µL/well. After adsorbing each antigen at 4°C overnight, the plates were blocked with blocking buffer (1% BSA, 3% sucrose, 0.1% ProClin300, in PBS) for 2 hours. Then, the culture supernatant of the hybridoma prepared above was added and incubated for 1 hour. After washing 4 times with a washing buffer (0.05% Tween 20 in PBS), a 5000-fold diluted POD-Goat anti-mouse IgGFcγ (Jackson Immuno Research, 115-036-071) was added to each well at 100 µL/well. After incubating for 30 minutes, each well was washed 4 times with wash buffer and colored with TMB solution (Nacalai, 05298). The color reaction was stopped with H₂SO₄, and the absorbance of each well was measured with a plate reader.

Thereby, the present inventors selected hybridoma strains (CIV09 strain, CIV13 strain) that could produce an anti-type IV collagen 7S domain monoclonal antibody (anti-7S domain antibody), capable of binding to the plate immobilized with the type IV collagen 7S fragments. The CIV09 strain and the CIV13 strain thus selected bind to both the pepsin-solubilized type IV collagen fragments and the type IV collagen 7S fragments. Meanwhile, the present inventors selected a hybridoma strain (CIV03 strain) that could produce an anti-type IV collagen monoclonal antibody, capable of binding to a plate immobilized with the pepsin-solubilized type IV collagen fragments, but not to a plate immobilized with the type IV collagen 7S fragments. Each hybridoma strain was cultured in a serum-free medium (Hybridoma SFM, Gibco), and purified from the obtained culture supernatant using a protein A column to obtain an antibody solution containing anti-type IV collagen 7S domain monoclonal antibody or anti-type IV collagen monoclonal antibody. The protein concentration of each antibody solution was determined by the absorbance at a wavelength of 280 nm.

### (3) Confirmation of reaction specificity of monoclonal antibody

The type IV collagen 7S fragments and the pepsin-solubilized type IV collagen fragments prepared in (1) above were used as antigens, each diluted with PBS to 1 µg/mL, and dispensed into a 96-well plate at 100 µL/well. After adsorbing each antigen at 4°C overnight, the plates were blocked with blocking buffer for 2 hours. Then, each antibody solution obtained in (2) above was serially diluted to 1000 ng/mL to 3.9 ng/mL, dispensed at 100 µL/well, and incubated for 1 hour. After washing 4 times with the washing buffer, the 5000-fold diluted POD-Goat anti-mouse IgGFcγ was added to each well at 100 µL/well. After incubating for 30 minutes, each well was washed 4 times with washing buffer and colored with TMB solution. The color reaction was stopped with H₂SO₄, and the absorbance of each well was measured with a plate reader.

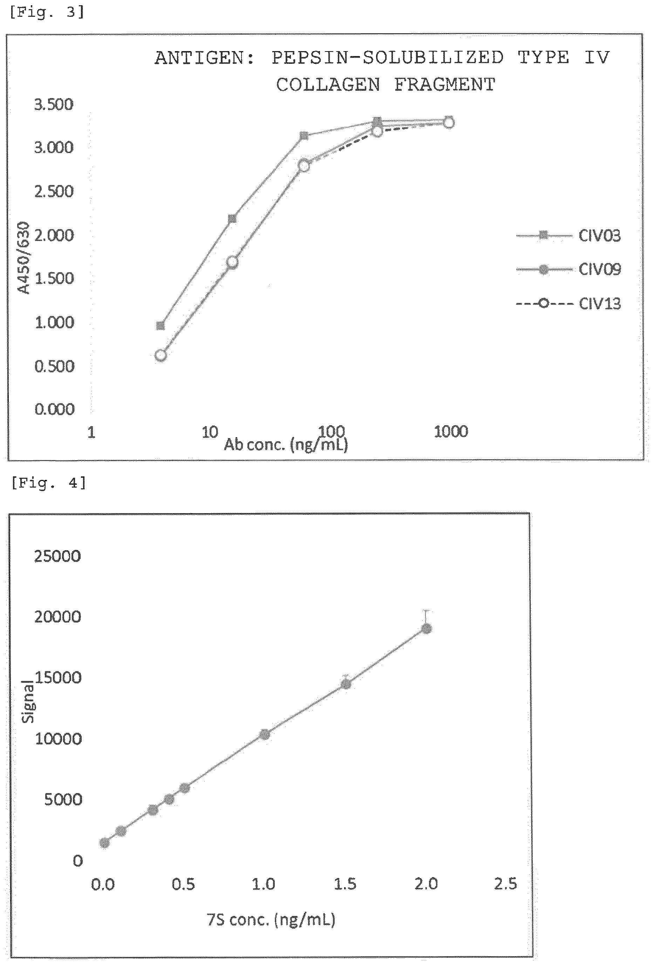

Fig. 2 shows the results showing the relationship between the concentration (Ab conc. (ng/mL)) and absorbance (ratio of wavelength 450 nm and 630 nm (A450/630), the same applies hereinafter) of each monoclonal antibody when the type IV collagen 7S fragments prepared in (1) above were used as an antigen, and Fig. 3 shows the relationship between the concentration (Ab conc. (ng/mL)) and absorbance of each monoclonal antibody when the pepsin-solubilized type IV collagen fragments were used as an antigen. We confirmed to have obtained monoclonal antibodies (CIV09, CIV13: anti-type IV collagen 7S domain monoclonal antibodies) capable of specifically binding to the type IV collagen 7S fragments obtained by the collagenase treatment, and a monoclonal antibody (CIV03: anti-type IV collagen monoclonal antibody) capable of binding to the pepsin-solubilized type IV collagen fragments but not to the type IV collagen 7S fragments.

### (4) Preparation of antibody-bound particles

Using a carboxyl-amine cross-linking agent (carbodiimide, Thermo-Fisher Scientific) and following the product manual, the anti-type IV collagen 7S domain monoclonal antibody CIV09 (hereinafter referred to as the "CIV09 antibody" in some cases) was chemically bonded to magnetic particles (manufactured by Fujirebio) to obtain CIV09 antibody-immobilized magnetic particles. Similarly, the anti-type IV collagen monoclonal antibody CIV03 (hereinafter referred to as the "CIV03 antibody" in some cases) was chemically bonded to magnetic particles to obtain CIV03 antibody-immobilized magnetic particles.

### (5) Preparation of labeled antibody

The anti-type IV collagen 7S domain monoclonal antibody CIV13 (hereinafter referred to as the "CIV13 antibody" in some cases) and alkaline phosphatase derived from bovine small intestine (manufactured by Oriental Yeast Co., Ltd.) were bound by the method of Yoshitake et al. (Yoshitake et al., J. Biochem. 1982, 92 (5), p. 1413-1424) to prepare an alkaline phosphatase-labeled CIV13 antibody. Specifically, first, as is usually carried out, the desalted CIV13 antibody and pepsin were mixed in 0.1 M citrate buffer solution (pH 3.5) and allowed to stand at 37°C for 1 hour for pepsin digestion. The reaction was stopped, and then gel filtration purification was carried out to obtain a CIV13 antibody with the Fc region removed therefrom. Then, 2-mercaptoethylamine hydrochloride was added to carry out thiolation. Further, this was desalted to obtain Fab' fragments of the CIV13 antibody.

N-(4-maleimide butyryloxy)-succinimide (GMBS)-treated alkaline phosphatase and the Fab' fragments of the CIV13 antibody were mixed and coupled. 2-Mercaptoethylamine hydrochloride and iodoacetamide were added to the coupling solution to terminate the reaction, thereby obtaining alkaline phosphatase-labeled CIV13 antibody (ALP-labeled CIV13 antibody).

### (6) Examination of salt concentration

First, 30 µL of a sample was added to 50 µL of particle suspensions having different salt concentrations (50 mM Tris, 1% BSA, 0.15 M to 1.5 M NaCl) containing 0.03% of CIV09 antibody-immobilized magnetic particles prepared in (4) above, and the mixture was reacted at 37°C for 8 minutes. The sample used was serum derived from a patient with liver cirrhosis, non-alcoholic steatohepatitis (liver disease), or liver cancer (patient specimen), serum derived from a healthy person (healthy person specimen), or type IV collagen 7S fragments (Type IV COL 7S), pepsin-solubilized type IV collagen fragments (Type IV COL), or collagenase partially digested type IV collagen fragments (partially digested Type IV COL) prepared in (1) above, each diluted with Tris buffer (50 mM Tris, 2% BSA, 0.15 M NaCl, pH 7.2) to 10 ng/mL. When the particle suspension contains 0.15 M, 0.3 M, 0.6 M, 1.0 M, 1.2 M, or 1.5 M NaCl, the salt concentration of the reaction system added with each sample is 0.15 M, 0.24 M, 0.43 M, 0.68 M, 0.80 M, or 0.99 M.

After the reaction, B/F separation was carried out with a magnet and washed with Lumipulse (registered trademark) washing solution (manufactured by Fujirebio), and then 50 µL of a labeled body fluid containing 1 µg/mL of the ALP-labeled CIV13 antibody prepared in (5) above (50 mM Bis-Tris, 600 mM NaCl, 2% BSA, 1 mM MgCl₂, 0.3 mM ZnCl₂) was added, and the mixture was reacted at 37°C for 8 minutes. After B/F separation with a magnet and washing with Lumipulse washing solution, 200 µL of Lumipulse (registered trademark) substrate solution (manufactured by Fujirebio) containing 3-(2'-spiroadamantane)-4-methoxy-4-(3"-phosphoryloxy) phenyl-1,2-dioxetane·disodium salt (AMPPD) was added, and an enzymatic reaction was carried out at 37°C for 4 minutes to measure the luminescence at a wavelength of 463 nm. Similarly, a standard solution (50 mM Tris, 0.15 M NaCl, 2% BSA, pH 7.2) containing the type IV collagen 7S fragments having a known concentration was measured to prepare a calibration curve. Using the prepared calibration curve, the concentration of the human type IV collagen fragments containing the 7S domain in each sample was calculated from the luminescence of each sample.

Table 1 to 3 present the results. In Tables 1 to 3, the salt concentration indicates the salt concentrations of the particle suspensions, and each value indicates the ratio (%) of the luminescence under respective condition to the luminescence when the salt concentration of the particle suspension is 0.15 M. Further, in Table 1, each value for patient specimen shows the average value of the specimens derived from 15 liver disease patients (liver disease average), and each value for healthy person specimen shows the average value of the specimens derived from 130 healthy persons (healthy person average). Meanwhile, in Table 3, the patient specimen and the healthy person specimen each shows the values for 5 of the corresponding specimens (specimens 1 to 5).

**[Table 1]**

| Salt Concentration | 0.15 M | 0.6 M | 1.0 M | 1.2 M | 1.5 M |
|---|---|---|---|---|---|
| Type IV COL 7S | 100% | 104% | 101% | 103% | 106% |
| Liver Disease Average | 100% | 90% | 90% | 91% | 90% |
| Healthy Person Average | 100% | 83% | 80% | 80% | 76% |

**[Table 2]**

| Salt Concentration | 0.6 M | 1.0 M | 1.2 M | 1.5 M |
|---|---|---|---|---|
| Type IV COL | 105% | 123% | 132% | 164% |
| Partially Digested Type IV COL | 104% | 114% | 118% | 117% |

**[Table 3]**

| Salt concentration | | 0.15 M | 0.3 M | 0.6 M | 1.0 M |
|---|---|---|---|---|---|
| TYPE IV COL 7S | | 100% | 101% | 104% | 105% |
| Patient Specimen 1 | | 100% | 94% | 106% | 104% |
| Patient Specimen 2 | | 100% | 93% | 104% | 104% |
| Patient Specimen 3 | | 100% | 97% | 110% | 106% |
| Patient Specimen 4 | | 100% | 96% | 105% | 106% |
| Patient Specimen 5 | | 100% | 91% | 99% | 98% |
| Healthy Specimen 1 | Person | 100% | 89% | 87% | 83% |
| Healthy Specimen 2 | Person | 100% | 87% | 84% | 79% |
| Healthy Specimen 3 | Person | 100% | 86% | 84% | 83% |
| Healthy Specimen 4 | Person | 100% | 89% | 86% | 83% |
| Healthy Specimen 5 | Person | 100% | 87% | 79% | 77% |

As shown in Tables 1 and 3, when the sample was a type IV collagen 7S fragment (Type IV COL 7S), no change in luminescence was observed depending on the salt concentration. Meanwhile, as shown in Table 3, when the salt concentration was set to 0.3 M (0.24 M as the salt concentration of the reaction system), the luminescence decreased in both the patient specimens and the healthy person specimens, and particularly in the healthy person specimens. Further, when the salt concentration was raised to 0.6 M to 1.0 M (0.43 M to 0.68 M as the salt concentration of the reaction system), the luminescence of the patient specimens increased, but for the healthy subject specimens, the luminescence decreased as the salt concentration increased, and this tendency was significantly larger than that of the patient specimens. As shown in Table 1, when the salt concentration was 0.6 M to 1.5 M (0.43 M to 0.99 M as the salt concentration of the reaction system), the luminescence in the healthy subject specimens was significantly low. This shows that non-specific reactions in healthy person specimens are reduced, especially at high salt concentrations.

Further, as shown in Tables 1 and 2, no change in luminescence was observed in the type IV collagen 7S fragments despite varying salt concentrations, whereas when the samples were the pepsin-solubilized type IV collagen fragments (Type IV COL) and the collagenase partially digested type IV collagen fragments (partially digested Type IV COL), at salt concentrations of 0.6 M (0.43 M as the salt concentration of the reaction system) or more, the luminescence increased as the salt concentration increased. The reason for this would be as follows. The 7S domain would be masked in fragments containing part or all of the TH domain other than the type IV collagen 7S fragments, contained in the pepsin-solubilized type IV collagen fragments and the collagenase partially digested type IV collagen fragments, and the mask came off as the salt concentration increased.

Thus, the specimens contained a wide variety of human type IV collagen fragments containing the 7S domain, as well as collagen fragments with low exposure due to a masked 7S domain, such as the TH domain-containing fragments contained in the pepsin-solubilized type IV collagen fragments and collagenase partially digested type IV collagen fragments, but increased salt concentrations led to a constant degree of exposure, making it possible to obtain more accurate measured values.

From the above, it was confirmed that a salt concentration of the reaction system set to, in particular, 0.35 M or more (for example, 0.43 M (salt concentration of particle suspension 0.6 M) or more) reduced the non-specific reaction especially in the healthy person specimens, in the sandwich immunoassay of the present invention using a combination of anti-type IV collagen 7S domain monoclonal antibody-immobilized magnetic particles and a labeled anti-type IV collagen 7S domain monoclonal antibody.

### (7) Examination of the sensitivity of the measurement system

As a sample, the type IV collagen 7S fragments prepared in (1) above were weight-diluted with a standard solution diluted solution to prepare 8 types of diluted series of standard solutions. The luminescence was measured in the same manner as in (6) above except that the prepared sample was used with the salt concentration of the particle suspension set to 0.6 M, and in addition, the concentration of the human type IV collagen fragments containing the 7S domain in each sample was calculated using the calibration curve. The measurement of each low-concentration standard solution (2.0 ng/mL or less) was repeated 20 times and the measurement of the others was carried out in duplicate.

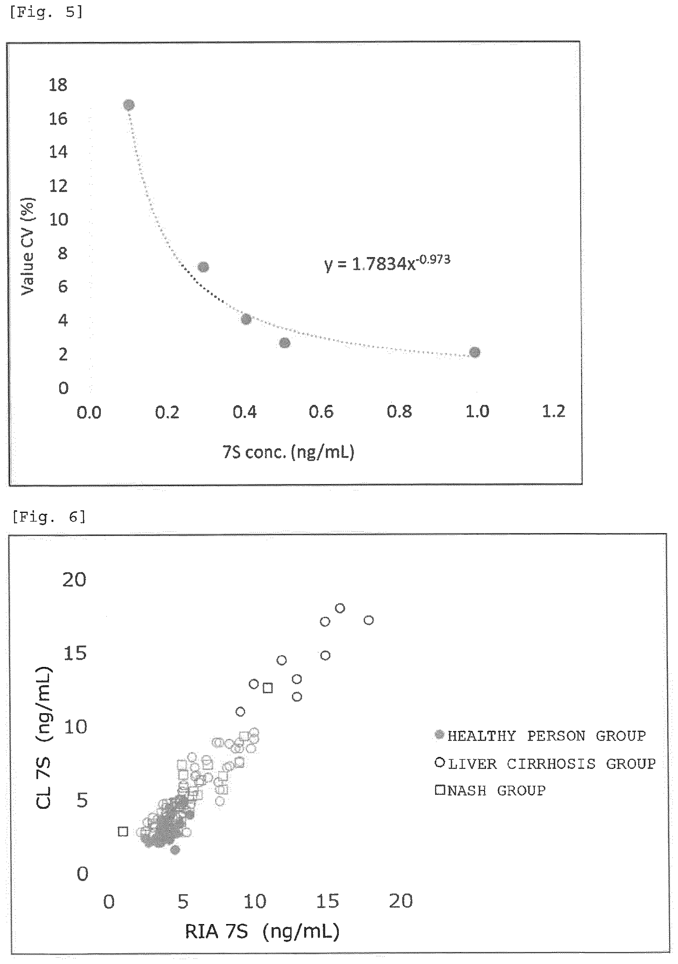

Fig. 4 shows the results showing the relationship between the concentration (7S conc. (Ng/mL)) and luminescence (Signal, average value) of the type IV collagen 7S fragments in the standard solution. Further, Fig. 5 shows the results showing the relationship between the actual concentration (7S conc. (ng/mL)) of the type IV collagen 7S fragments in the standard solution and the coefficient of variation (Value CV(%), average value) of the concentration (quantitative value) of the human type IV collagen fragments containing the 7S domain quantified by the calibration curve. The limit of detection was set as the concentration of a low-concentration standard solution, at which the average value of the measurement count of 0 ng/mL + 2SD was less than the average value of the luminescence (Signal) of the low-concentration standard solution - 2SD (SD: standard deviation). In addition, the limit of quantification was a concentration at which the coefficient of variation (Value CV (%)) was 10% or less. From Fig. 4, the detection limit was 0.1 ng/mL or less, and from Fig. 5, the limit of quantification was 0.3 ng/mL or less.

### <Comparison with the conventional method (Comparative Examples)>

### (Example 2)

Samples used were 30 cases of serum derived from healthy persons (healthy person group), 56 cases of serum derived from patients with liver cirrhosis (liver cirrhosis group), and 38 cases of serum derived from patients with NASH (NASH group). The luminescence was measured in the same manner as in (7) of Example 1 above except that each of these specimens was used, and the calibration curve was used to calculate the concentration of fragments containing the type IV collagen 7S domain in each specimen.

### (Comparative Example 1)

The same specimens as in Example 2 were measured using Type IV Collagen 7S Kit (manufactured by DENIS Pharma, hereinafter referred to as the "RIA kit" in some cases), a radioimmunoassay using anti-human type IV collagen rabbit polyclonal antibody, according to the package insert to calculate the concentration of fragments containing the type IV collagen 7S domain in each specimen. According to the RIA kit, fragments containing the type IV collagen 7S domain in each specimen ("Type IV Collagen 7S" in the RIA kit, target substance) react with the anti-human type IV collagen rabbit polyclonal antibody to form a complex of fragments containing the type IV collagen 7S domain and anti-human type IV collagen rabbit polyclonal antibody (complex 1). Then, when iodide fragments containing the type IV collagen 7S domain ("Type IV Collagen 7S (¹²⁵I)" in the RIA kit) (labeled antigen) are added, the anti-human type IV collagen rabbit polyclonal antibody that failed to bind to the target substance in the specimen reacts with the labeled antigen to form a complex of labeled antigen and anti-human type IV collagen rabbit polyclonal antibody (complex 2). When anti-rabbit γ-globulin goat serum (goat antibody) is added thereto, the goat antibody reacts with the complex 2 to form a complex of labeled antigen, anti-human type IV collagen rabbit polyclonal antibody, and goat antibody, followed by precipitation. Thereby, the concentration of the target substance (fragments containing the type IV collagen 7S domain) can be determined by measuring the radioactivity of the precipitates after removing the unreacted labeled antigen.

### (Correlation)

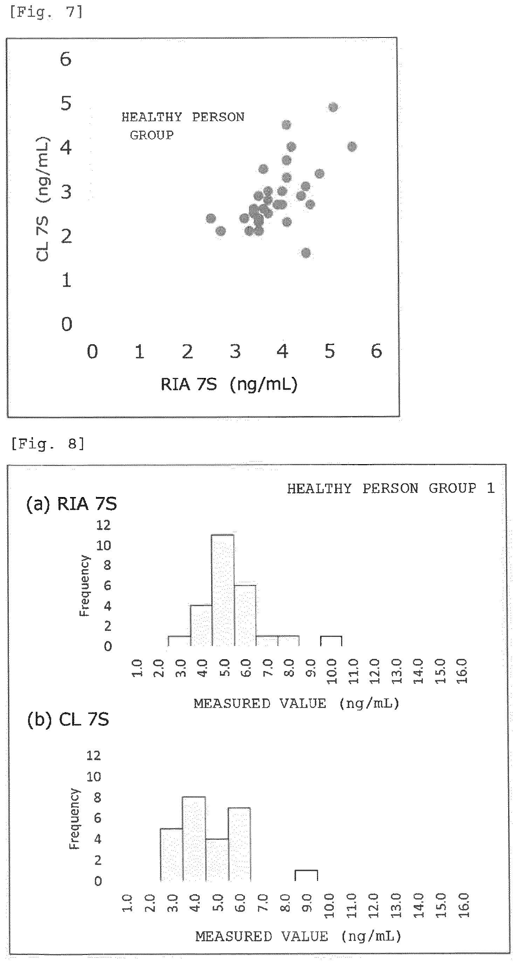

Fig. 6 shows the correlation between the measurement results of Example 2 (CL 7S: concentration of fragments containing type IV collagen 7S domain in the specimen (ng/mL)) and the measurement results of Comparative Example 1 (RIA 7S: concentration of fragments containing type IV collagen 7S domain in the specimen (ng/mL)). In addition, Fig. 7 shows the correlation only for the healthy person group. Note that Fig. 6 does not include the following LC51 and LC28 (that is, liver cirrhosis group: 54 cases). As shown in Fig. 6, the correlation between the measurement results by the sandwich immunoassay of the present invention using a combination of anti-type IV collagen 7S domain monoclonal antibody-immobilized magnetic particles and a labeled anti-type IV collagen 7S domain monoclonal antibody (CL 7S) and the measurement results by the RIA kit (RIA 7S) was very high with a correlation coefficient of 0.947. However, as shown in Fig. 7, in the healthy person group alone, the correlation coefficient was as low as 0.581, and it was found that the measured values for the healthy person specimens were clearly reduced according to the sandwich immunoassay of the present invention. That is, according to the sandwich immunoassay using the monoclonal antibody of the present invention, the measured values for the liver cirrhosis group and the NASH group do not decrease, but only the measured values of the healthy person group can be decreased, indicating that the patient group and the healthy person group can be divided with higher accuracy than the conventional RIA kit.

### (Evaluation of diagnostic performance 1)

### (Example 3)

As samples, 25 cases of serum derived from healthy persons (healthy person group 1) and 30 cases of serum derived from healthy persons (healthy person group 1) were used. The luminescence was measured in the same manner as in Example 2 above except that each of these specimens was used, and the concentration of fragments containing the type IV collagen 7S domain in each specimen was calculated using a calibration curve.

### (Comparative Example 2)

The concentration of fragments containing the type IV collagen 7S domain in each specimen was calculated using the RIA kit in the same manner as in Comparative Example 1 except that the same specimens as in Example 3 were used.

### [Distribution]

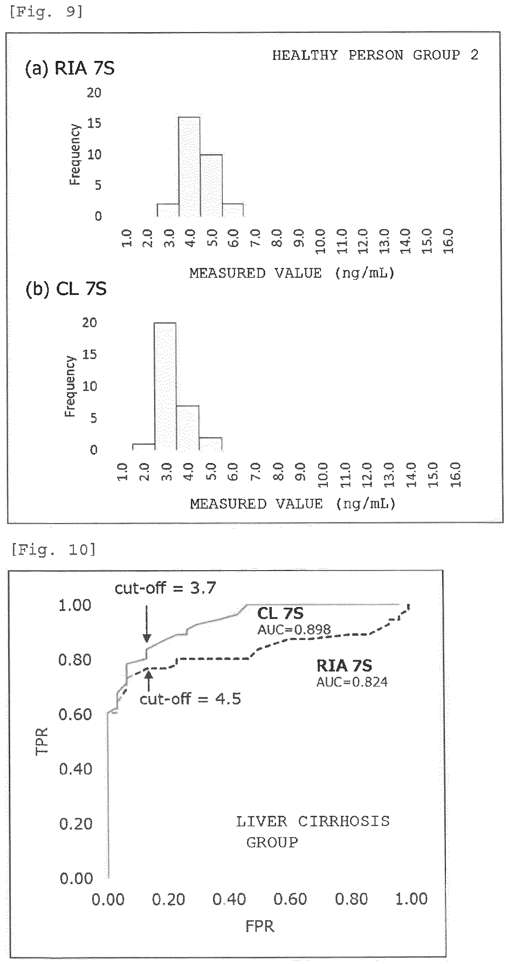

Fig. 8 shows the distribution of the measurement results (measured values: concentrations (ng/mL) of fragments containing the type IV collagen 7S domain in the specimens) of Example 3 ((b) CL 7S) and Comparative Example 2 ((a) RIA 7S) for the healthy person group 1. Further, for the healthy person group 2, Fig. 9 shows the distribution of the measurement results of Example 3 ((b) CL 7S) and Comparative Example 2 ((a) RIA 7S).

As shown in Figs. 8 to 9, in the sandwich immunoassay using the monoclonal antibody of the present invention, it was confirmed that in both healthy person groups, the measured values were clearly lower than those of the RIA kit, and the false positive rate in the healthy person groups could be reduced.

### (Evaluation of diagnostic performance 2)

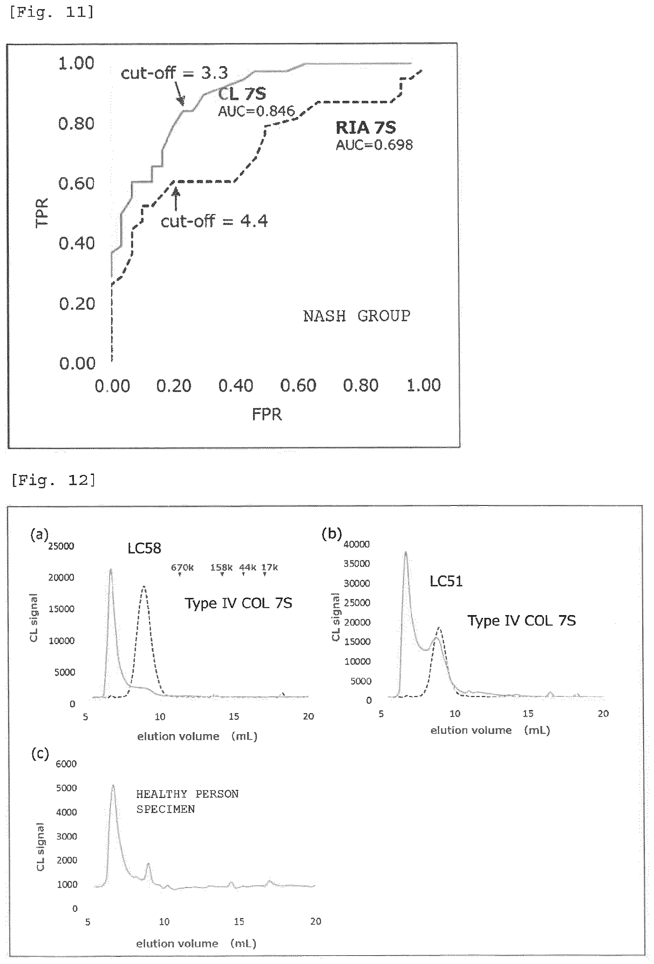

The diagnostic accuracy of Example 2 (CL 7S) and Comparative Example 1 (RIA 7S) was compared by ROC (Receiver Operating Characteristic) analysis. Fig. 10 shows an ROC curve of the liver cirrhosis group (FPR (false positive rate, 1-specificity)-TPR (positive rate, sensitivity) curve), and Fig. 11 shows an ROC curve of the NASH group. As shown in Fig. 10, in the liver cirrhosis group, the AUC value in Comparative Example 1 (RIA 7S) was 0.824, whereas the AUC value in Example 2 (CL 7S) was 0.898, indicating that diagnostic accuracy of liver cirrhosis was particularly high in the sandwich immunoassay using the monoclonal antibody of the present invention. Further, as shown in Fig. 11, also in the NASH group, the AUC value in Comparative Example 1 (RIA 7S) was 0.698, whereas the AUC value in Example 2 (CL 7S) was 0.846, indicating that the diagnostic accuracy of NASH was particularly high in the sandwich immunoassay of the present invention.

### (Measurable form of type IV collagen)

In the correlation between Example 2 and Comparative Example 1 above, among the specimens in the liver cirrhosis group, deviated specimens (LC51, LC28) whose measured values in Comparative Example 1 were much lower than those measured in Example 2 were found. Therefore, the forms of type IV collagen contained in the specimens of the liver cirrhosis group (LC51) with a large deviation, the specimens of the liver cirrhosis group (LC58) with a relatively small deviation of the measured values, and the specimens of the healthy person group (healthy person specimens) were compared.

### (Example 4)

First, 500 µL of each specimen was applied to Superose 6 FPLC 10/300 equilibrated with a fractionation buffer solution (50 mM Tris, 150 mM NaCl, 0.05% CHAPS, 0.09% NaN3, pH 7.2), and the fractionation buffer solution was developed at 0.5 mL/min, and gel-filtered. The resultant was fractionated to 0.25 mL per fraction to prepare a sample, and the luminescence (CL signal) of each fraction was measured in the same manner as in (7) of Example 1 above. Further, as a control sample, the type IV collagen 7S fragments prepared in (1) above were also fractionated and measured in the same manner.

### (Comparative Example 3)

As a comparison target, the luminescence of the fractions of each sample was measured in the same manner as in Example 4 except that the CIV03 antibody-immobilized magnetic particles were used as the antibody-immobilized magnetic particles.

### [Results]

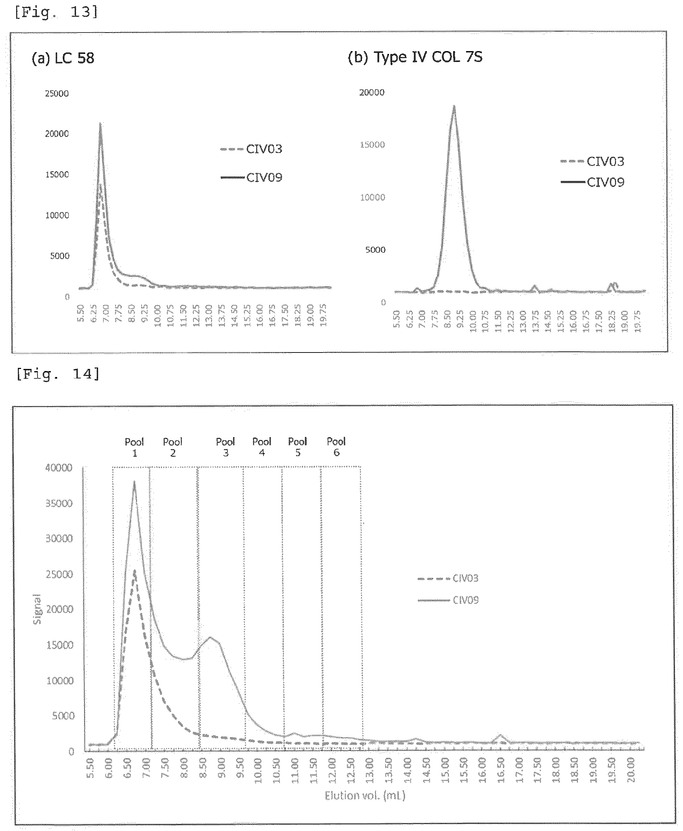

Fig. 12(a) shows the luminescence of each fraction of the specimen LC58 (solid line), Fig. 12(b) shows the luminescence of each fraction of the specimen LC51 (solid line), and Fig. 12(c) shows the luminescence of each fraction of the healthy person specimens (solid line), when the CIV09 antibody-immobilized magnetic particles were used (Example 4). Figs. 12(a) to 12(b) also show the luminescence (Type IV COL 7S, dotted line) of each fraction of the type IV collagen 7S fragments. Further, Fig. 13(a) shows the luminescence of each fraction of the specimen LC58, and Fig. 13(b) shows the luminescence of each fraction of the type IV collagen 7S fragments, when CIV09 antibody-immobilized magnetic particles are used (Example 4, solid line) or when CIV03 antibody-immobilized magnetic particles are used (Comparative Example 3, dotted line).

As shown in Fig. 12(a), in the specimen LC58, a signal was detected only in the high molecular weight fraction. Meanwhile, as shown in Fig. 12 (b), in the specimen LC51 with a large deviation between Example 2 and Comparative Example 1, a signal was detected in the high molecular weight fraction as well as in the molecular weight fraction corresponding to the type IV collagen 7S fragments obtained by collagenase treatment. Further, as shown in Figs. 13(a) and 13(b), in the case of measurement using CIV03 antibody-immobilized magnetic particles, no signal could be detected from any of the fractions in the type IV collagen 7S fragments (Fig. 13(b)), and likewise, no signal could be detected in the fractions corresponding to the type IV collagen 7S fragments in the specimen LC58 (Fig. 13(a)) .

That is, in order to comprehensively measure the "fragments containing the type IV collagen 7S domain" in the specimens derived from patients such as liver cirrhosis, including the small molecule type IV collagen 7S fragments, it has been shown that a sandwich immunoassay using a monoclonal antibody capable of binding to pepsin-solubilized type IV collagen fragments but not to type IV collagen 7S fragments as at least a capture antibody or labeled antibody is not sufficient. Therefore, it has been shown that the sandwich immunoassay of the present invention using a monoclonal antibody specific for the type IV collagen 7S domain for both the capture antibody and the labeled antibody makes it possible to more accurately measure fragments containing the type IV collagen 7S domain in specimens derived from patients such as liver cirrhosis.

### (Quantitative evaluation)

The dilution linearity was examined for the specimens whose measured values deviated between Example 2 and Comparative Example 1 described above. If a measured value has been obtained by a specific reaction, when the measured value is diluted, a low measured value is obtained according to the dilution rate, so that the dilution linearity is recognized.

### (Example 5)

As samples, each specimen of the liver cirrhosis group was diluted 5-fold or 10-fold with a standard solution diluted solution (50 mM Tris, 0.15 M NaCl, 2% BSA, pH 7.2). The luminescence was measured in the same manner as in Example 2 above except that each of these specimens was used, and the concentration of fragments containing the type IV collagen 7S domain in each specimen was calculated using a calibration curve.

### (Comparative Example 4)

The concentration of fragments containing the type IV collagen 7S domain in each specimen was calculated using the RIA kit in the same manner as in Comparative Example 1 except that the same specimens as in Example 5 were used.

### [Results]

As for LC27, LC28, LC37, LC51, and LC58, including LC51 and LC28 with a large deviation between Example 2 and Comparative Example 1 among the specimens in the liver cirrhosis group, Table 4 below shows the measured values and their recovery rates (measured value × dilution rate/measured value when dilution rate is 1 × 100 (%)) in Example 5 (CL 7S) and Comparative Example 4 (RIA 7S).

**[Table 4]**

| | | RIA 7S | | CL 7S | |
|---|---|---|---|---|---|
| | Dilution Rate | Measured Value (ng/mL) | Recovery Rate | Measured Value (ng/mL) | Recovery Rate |
| LC27 | 1 | 16.0 | | 15.2 | |
| | 10 | 2.0 | 125% | 1.5 | 99% |
| LC28 | 1 | 18.0 | | 24.6 | |
| | 5 | 6.2 | 172% | 5.0 | 102% |
| LC37 | 1 | 15.0 | | 13.3 | |
| | 5 | 4.3 | 143% | 2.7 | 102% |
| | 10 | 1.5 | 100% | 1.3 | 98% |
| LC51 | 1 | 17.0 | | 38.7 | |
| | 5 | 10.0 | 294% | 8.3 | 107% |
| LC58 | 1 | 14.0 | | 15.4 | |
| | 5 | 3.3 | 118% | 3.1 | 101% |
| | 10 | < 1.0 | ND | 1.6 | |

As shown in Table 4, in all the specimens, the recovery rate was almost 100% in the sandwich immunoassay using the monoclonal antibody of the present invention, and the dilution linearity (quantitativity) could be confirmed, but dilution linearity was not obtained with the RIA kit.

### (Analysis of measurement value deviation specimens)

### (Example 6)

The specimen LC51 with the largest deviation in measured values between Example 2 and Comparative Example 1 was gel-filtered in the same manner as in the above (Measurable form of type IV collagen), and the luminescence (Signal) of each fraction was measured. In addition, the luminescence of each fraction was measured in the same manner as this except that CIV03 antibody-immobilized magnetic particles were used as the control antibody-immobilized magnetic particles. Further, each of the obtained fractions was divided into pools 1 to 6, and the luminescence (Signal) of each pool was measured in the same manner as in (7) of Example 1. Meanwhile, the specimen LC51 before gel filtration was also measured in the same manner and used as a direct measured value.

### (Comparative Example 5)

The luminescence of each pool was measured using the RIA kit in the same manner as in Comparative Example 1 except that the same pools as in Example 6 were used. Meanwhile, the specimen LC51 before gel filtration was also measured in the same manner and used as a direct measured value.

### [Results]

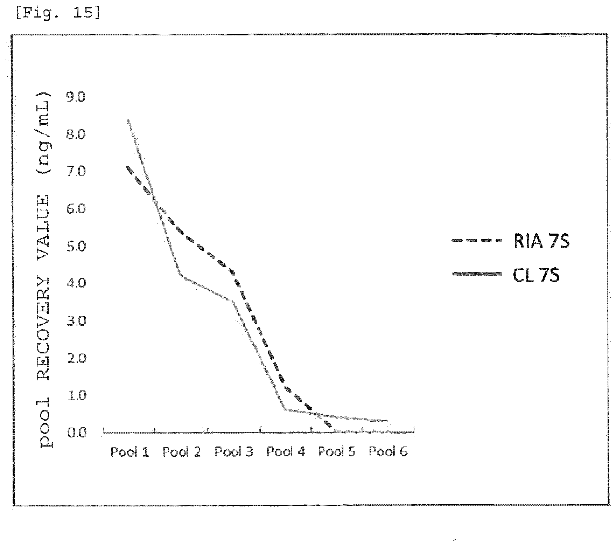

Fig. 14 shows, for the specimen LC51, the luminescence of each fraction when CIV09 antibody-immobilized magnetic particles were used (Example 6, solid line), and the luminescence of each fraction when CIV03 antibody-immobilized magnetic particles were used (dotted line). In addition, the concentration of fragments containing the type IV collagen 7S domain in each pool was calculated as the pool recovery value (ng/mL) from the measured value of each pool. Fig. 15 shows, for the specimen LC51, each pool recovery value of the sandwich immunoassay using a monoclonal antibody (Example 6, CL 7S, solid line), and each pool recovery value when the RIA kit is used (Comparative Example 5, RIA 7S, dotted line).

Further, the total of the pool recovery values of pools 1 to 6 was redefined as the recovery value, and the recovery rate was calculated from each direct measured value using the following formula: recovery rate (%) = recovery value/direct measured value × 100. As a result, the recovery rate in the sandwich immunoassay using the monoclonal antibody of the present invention (Example 6, CL 7S) was 87%. Meanwhile, in the RIA kit (Comparative Example 5, RIA), the direct measured value was low, and the recovery rate was 237%, which was much higher than 100%. It is inferred from the above that the CIV03 antibody and the anti-human type IV collagen polyclonal antibody used in the RIA kit are greatly affected by the substances present in the samples and the sites other than the 7S domain of type IV collagen, and it is probable that the correct values were not given in the measurements using these. Meanwhile, it was shown that the sandwich immunoassay using the monoclonal antibody of the present invention was able to specifically detect fragments containing the type IV collagen 7S domain.

### [Industrial Applicability]

The present invention makes it possible to provide a measurement method which makes it possible to reduce non-specificity for a sample derived from a healthy person (negative specimen) without reducing the specificity for a sample derived from a patient (positive specimen) and to measure fragments containing a human type IV collagen 7S domain in a sample with high accuracy, and a kit for use therein.

Fragments containing the human type IV collagen 7S domain in blood are mainly used as biomarkers for the diagnosis of liver cirrhosis and non-alcoholic steatohepatitis (NASH). By using the present invention as an aid to diagnosis, it is possible to improve the diagnostic accuracy and sufficiently reduce the non-specificity to specimens derived from healthy persons. Therefore, it is possible to screen and differentiate patients in the early stages of liver fibrosis.

## Claims

1. A measurement method comprising: measuring a fragment containing a human type IV collagen 7S domain in a sample by sandwich immunoassay, using a capture antibody in which a first monoclonal antibody capable of binding specifically to the human type IV collagen 7S domain is immobilized on a carrier and a labeled antibody in which a second monoclonal antibody capable of binding specifically to the human type IV collagen 7S domain binds to a labeling substance.

2. The measurement method according to claim 1, comprising:
a capturing step of contacting the sample with the capture antibody to capture the fragment containing the human type IV collagen 7S domain using the capture antibody; and
after the capturing step, a labeling step of contacting the labeled antibody with the fragment containing the human type IV collagen 7S domain captured by the capture antibody to label the fragment containing the human type IV collagen 7S domain captured by the capture antibody.

3. The measurement method according to claim 1 or 2, wherein a salt concentration of a reaction system of the fragment containing the human type IV collagen 7S domain and the first monoclonal antibody is 0.35 M or more.

4. A kit for use in the measurement method according to any one of claims 1 to 3, comprising:
a first monoclonal antibody capable of binding specifically to the human type IV collagen 7S domain and a carrier capable of binding to the first monoclonal antibody; and
a second monoclonal antibody capable of binding specifically to the human type IV collagen 7S domain and a labeling substance capable of binding to the second monoclonal antibody.

5. The kit according to claim 4, comprising:
a capture antibody in which the first monoclonal antibody is immobilized on the carrier; and
a labeled antibody in which the second monoclonal antibody is bound to the labeling substance.
